(19) Europäisches Patentamt
European Patent Office
Office européen des brevets

(11) **EP 3 752 044 B1**

(12) **EUROPÄISCHE PATENTSCHRIFT**

(45) Veröffentlichungstag und Bekanntmachung des Hinweises auf die Patenterteilung:
**07.06.2023** **Patentblatt 2023/23**

(21) Anmeldenummer: **19715389.3**

(22) Anmeldetag: **12.02.2019**

(51) Internationale Patentklassifikation (IPC):
***A61B 1/06*** *(2006.01)* ***A61B 1/04*** *(2006.01)*

(52) Gemeinsame Patentklassifikation (CPC):
**A61B 1/0638; A61B 1/043; A61B 1/0646; A61B 1/0676; A61B 1/0684**

(86) Internationale Anmeldenummer:
**PCT/DE2019/200012**

(87) Internationale Veröffentlichungsnummer:
**WO 2019/158168 (22.08.2019 Gazette 2019/34)**

(54) **MEDIZINISCH-ENDOSKOPISCHES INSTRUMENT**

MEDICAL ENDOSCOPIC INSTRUMENT

INSTRUMENT MÉDICAL ENDOSCOPIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.02.2018 DE 102018202243**

(43) Veröffentlichungstag der Anmeldung:
**23.12.2020 Patentblatt 2020/52**

(73) Patentinhaber: **Richard Wolf GmbH**
**75438 Knittlingen (DE)**

(72) Erfinder:
• **WEBER, Bernd Claus**
**76228 Karlsruhe (DE)**
• **HANDTE, René**
**71665 Vaihingen/Enz (DE)**

• **IMPELLIZZERI, Alessandro**
**75173 Pforzheim (DE)**
• **DOLT, Martin**
**75438 Knittlingen (DE)**

(74) Vertreter: **Patentanwälte Vollmann Hemmer Lindfeld**
**Partnerschaft mbB**
**Wallstraße 33a**
**23560 Lübeck (DE)**

(56) Entgegenhaltungen:
**EP-A1- 1 745 736** **WO-A1-2012/069542**
**DE-A1-102005 015 374** **US-A1- 2010 321 772**
**US-A1- 2011 257 484** **US-A1- 2013 012 815**
**US-A1- 2013 070 071**

**Beschreibung**

Die vorliegende Offenbarung betrifft ein medizinisch-endoskopisches Instrument mit einem distalen länglichen Einführabschnitt zum minimal-invasiven Einführen in einen menschlichen oder tierischen Körper.

Es ist bekannt, Endoskope dazu zu nutzen, um Videoaufnahmen vom Inneren eines menschlichen oder tierischen Körpers zu Zwecken der medizinischen Diagnose und/oder Therapie zu machen. Dabei ist es üblich, das Körperinnere mit einer Lichtquelle auszuleuchten und über einen CCD-Sensor eine Bildaufnahme durchzuführen. Da sich die Spektralempfindlichkeit eines CCD-Sensors von der eines menschlichen Auges unterscheidet, wird üblicherweise im Stand der Technik im Bildpfad vor dem CCD-Sensor ein Korrekturfilter gesetzt, um einen natürlichen Farbeindruck des aufgenommenen Bildes zu erzeugen. CCD-Sensoren sind üblicherweise insbesondere im roten und infraroten Wellenlängenbereich empfindlicher als das Auge, sodass die eingesetzten Korrekturfilter in diesem Wellenlängenbereich besonders stark dämpfen. Nachteilig dabei ist allerdings, dass wegen des Korrekturfilters signifikante Anteile der Lichtleistung, die in das Körperinnere eingekoppelt und dort in Wärme umgesetzt werden, nicht für die Bildaufnahme des CCD-Sensors genutzt werden.

Zur besseren Nutzung bzw. Einsparung der eingekoppelten Lichtleistung schlägt die WO 95/17845 vor, ein dichroitisches Korrekturfilter nicht im Bildpfad vor dem CCD-Sensor zu platzieren, sondern vor eine externe Lichtquelle oder in einem Lichtleitsystem des Endoskops anzuordnen. Es wird also Licht, das der CCD-Sensor nicht aufnehmen soll, gar nicht erst in den Körper eingekoppelt. Damit wird das Gewebe gegen die Einkopplung unnötiger Wärme- und Lichtleistung geschützt.

Das aus der WO 95/17845 bekannte endoskopische Videosystem ist allerdings nicht dazu geeignet, wahlweise für die Weißlicht-Endoskopie und für die Fluoreszenz-Endoskopie eingesetzt zu werden. Im Gegensatz zur Weißlicht-Endoskopie kommt es bei der Fluoreszenz-Endoskopie, die beispielsweise für die Detektion und Lokalisierung von prä- und frühmalignem Gewebe eingesetzt wird, nicht auf eine natürliche Echtfarb-Darstellung des Gewebes an, sondern auf eine Fluoreszenzanregung, mit der sich pathologisches Gewebe von gesundem Gewebe unterscheiden lässt. Dabei kann das mittels Lichtstrahlung angeregte pathologische Gewebe selbst oder eine auf pathologisches Gewebe hinweisende Bakterienansammlung spezifisch fluoreszieren und so gegenüber dem umliegenden gesunden Gewebe erkennbar lokalisiert werden. Die Fluoreszenz-Endoskopie kann beispielsweise im Rahmen einer photodynamischen Diagnose (PDD) und/oder photodynamischen Therapie (PDT) mittels eines Photosensibilisators bzw. Markerstoffs (z.B. Chlorin e6) durchgeführt werden, der sich selektiv an pathologischem Gewebe anreichert. In der US 2013/0012815 A1 ist eine endoskopische Kapsel mit LED-Anregungslicht zur Fluoreszenzspektroskopie beschrieben. Die WO 2012/069542 A1 beschreibt ein Endoskop mit einer distalen LED und vorgelagertem optischen Konverter und Tiefpassfilter.

Die vorliegende Offenbarung stellt ein medizinisch-endoskopisches Instrument bereit, das wahlweise für die Fluoreszenz-Endoskopie und für die Weißlicht-Endoskopie eingesetzt werden kann und gleichwohl das Gewebe gegen die Einkopplung nicht genutzter Wärme- und Lichtleistung schützt, indem es die eingekoppelte Lichtleistung für den jeweiligen Verwendungszweck besser nutzt.

Gemäß der vorliegenden Offenbarung wird ein medizinisch-endoskopisches Instrument mit einem distalen länglichen Einführabschnitt zum minimal-invasiven Einführen in einen menschlichen oder tierischen Körper bereitgestellt, wobei der Einführabschnitt mindestens eine erste licht-emittierende Diode (LED), eine zweite LED und einen Bildsensor aufweist, wobei die erste LED, die zweite LED und der Bildsensor in eine gemeinsame Blickrichtung ausgerichtet sind. Die erste LED weist dabei ein für eine Fluoreszenz-Endoskopie geeignetes erstes Leuchtspektrum und die zweite LED ein für eine Weißlicht-Endoskopie geeignetes zweites Leuchtspektrum auf, wobei ein Lichtfilter in Blickrichtung vor der zweiten LED angeordnet ist. Das Lichtfilter weist dabei ein Transmissionsspektrum auf, wobei die zweite LED dazu ausgestaltet ist, gemäß dem zweiten Leuchtspektrum in einem ersten Wellenlängenbereich im Mittel intensiver zu strahlen als in einem zweiten Wellenlängenbereich, und wobei das Lichtfilter dazu ausgestaltet ist, von der zweiten LED ausgestrahltes Licht gemäß dem Transmissionsspektrum im zweiten Wellenlängenbereich im Mittel weniger durchzulassen als im ersten Wellenlängenbereich. Erfindungsgemäß ist die erste LED gegenüber der zweiten LED bezüglich der Blickrichtung nach vorne versetzt angeordnet.

Mit "Leuchtspektrum" sei hier eine Intensitätsverteilung $I(\lambda)$ des Lichts in Abhängigkeit von der Wellenlänge $\lambda$ des Lichts gemeint. Die mittlere Intensität in einem Wellenlängenbereich zwischen einer Wellenlänge $\lambda_1$ und einer

$$\bar{I} = \int_{\lambda_1}^{\lambda_2} \frac{I(\lambda)}{\lambda_2 - \lambda_1} d\lambda$$

Wellenlänge $\lambda_2$ sei hier definiert als                . Mit "Transmissionsspektrum" sei hier eine Verteilung der prozentualen Lichtdurchlässigkeit $T(\lambda)$ in Abhängigkeit von der Wellenlänge $\lambda$ des Lichts gemeint. Die mittlere Transmission bzw. Durchlässigkeit in einem Wellenlängenbereich zwischen einer Wellenlänge $\lambda_1$ und einer Wellenlänge $\lambda_2$ sei hier

$$\bar{T} = \int_{\lambda_1}^{\lambda_2} \frac{T(\lambda)}{\lambda_2 - \lambda_1} d\lambda.$$

definiert als                . Erstreckt sich also der erste Wellenlängenbereich von einer Wellenlänge $\lambda_1$ zu einer

Wellenlänge $\lambda_2$, und der zweite Wellenlängenbereich von einer Wellenlänge $\lambda_3$ zu einer Wellenlänge $\lambda_4$, so ergibt sich

$$\overline{I_1} = \int_{\lambda_1}^{\lambda_2} \frac{I_1(\lambda)}{\lambda_2 - \lambda_1} d\lambda > \overline{I_2} = \int_{\lambda_3}^{\lambda_4} \frac{I_2(\lambda)}{\lambda_4 - \lambda_3} d\lambda \quad \text{und} \quad \overline{T_1} = \int_{\lambda_1}^{\lambda_2} \frac{T_1(\lambda)}{\lambda_2 - \lambda_1} d\lambda > \overline{T_2} = \int_{\lambda_3}^{\lambda_4} \frac{T_2(\lambda)}{\lambda_4 - \lambda_3} d\lambda.$$

. Die mittlere Intensität $\overline{I_1}$ der zweiten LED im ersten Wellenlängenbereich ist also größer als die mittlere Intensität $\overline{I_2}$ der zweiten LED im zweiten Wellenlängenbereich. Ebenso ist die mittlere Transmission bzw. Durchlässigkeit $\overline{T_1}$ des Lichtfilters im ersten Wellenlängenbereich größer als die mittlere Transmission bzw. Durchlässigkeit $\overline{T_2}$ des Lichtfilters im zweiten Wellenlängenbereich. Sollten die Funktionen $I(\lambda)$ und/oder $T(\lambda)$ für bestimmte Wellenlängen bzw. Spektrallinien im ersten und/oder zweiten Wellenlängenbereich nicht lokal integrierbar sein, so sind solche Wellenlängen bzw. Spektrallinien bei der Mittelung zu ignorieren. Das Leuchtspektrum der zweiten LED und das Transmissionsspektrum des Lichtfilters ergänzen sich also bei der Unterdrückung des Lichts im zweiten, vorzugsweise roten und infraroten, Wellenlängenbereich. Der erste Wellenlängenbereich kann sich von einem kurzwelligen Ende (z.B. $\lambda_1 = 400$ nm) im sichtbaren Spektrum zum kurzwelligen Ende (z.B. $\lambda_2 = \lambda_3 = 550$ nm) des zweiten Wellenlängenbereichs erstrecken, von dem sich der zweite Wellenlängenbereich sich bis zu einem langwelligen Ende (z.B. $\lambda_4 = 700$ nm) im sichtbaren Spektrum erstreckt. Vorzugsweise grenzen der erste Wellenlängenbereich und der zweite Wellenlängenbereich aneinander und sind gleich groß. Der erste Wellenlängenbereich liegt vorzugsweise unterhalb des zweiten Wellenlängenbereichs.

Die zwei LEDs sind als Lichtquellen im Einführabschnitt angeordnet, um Licht "in situ" im Körper zu erzeugen, sodass es keiner externen Lichtquelle und keines Lichtleitsystems bedarf. Mit der ersten LED kann die Fluoreszenz-Endoskopie betrieben werden und mit der zweiten LED die Weißlicht-Endoskopie. Der Bildsensor, beispielsweise ein CCD-Sensor oder CMOS-Sensor, kann wahlweise für die Fluoreszenz-Endoskopie und die Weißlicht-Endoskopie genutzt werden und bedarf keines Korrekturfilters in Form eines Kurzpassfilters im Bildpfad, der die für die Bildgebung nutzbare Lichtleistung verringerte. Mit dem hierin offenbarten Instrument kann die Fluoreszenz-Endoskopie im Rahmen einer PDD und/oder PDT durchgeführt werden. Bevorzugte Ausführungsformen des Instruments können jedoch vornehmlich für die PDD ausgelegt sein, wenn beispielsweise die zweite LED ein kurzwelliges, zweites Leuchtspektrum hat, um effizient Fluoreszenz anzuregen. Für eine PDT ist ggf. ein langwelligeres, zweites Leuchtspektrum mit höherer Eindringtiefe in das Gewebe effektiver.

Allerdings bietet das hier offenbarte Instrument weitere Vorteile. Zum einen nehmen die LEDs lateral weniger Platz ein als Lichtleiter, sodass sie im sehr begrenzten Bauraum des Einführabschnitts mit gleicher Blickrichtung wie der Bildsensor neben diesem platziert werden können. Andererseits kann mit den LEDs jeweils mit weniger Aufwand, beispielsweise ohne Streulinse, und mit weniger optischen Verlusten ein größerer Raumwinkel mit ausreichender Helligkeit ausgeleuchtet werden als mit einem Lichtleiter. Das Leuchtspektrum der zweiten LED hat gegenüber dem Leuchtspektrum einer Halogen-, Xenon-, Halid- oder sonstigen Metall-Dampflampe bereits in dem zweiten Wellenlängenbereich, vorzugsweise im roten und infraroten Wellenlängenbereich, eine geringere Intensität, sodass das Lichtfilter für das LED-Licht dünner ausgestaltet werden kann als wenn Licht einer Halogen-, Xenon-, Halid- oder sonstigen Metall-Dampflampe gefiltert werden müsste. Durch den dünneren Lichtfilter kann die zweite LED weiter "vorn" in Blickrichtung an einer Außenfläche des Einführabschnitts, vorzugsweise dessen Stirnfläche, angeordnet werden, wodurch sich ein "Schlüssellocheffekt" bzw. "Tunnelblick-Effekt" verringert und der ausgeleuchtete Raumwinkel vergrö-ßert.

Die "Blickrichtung" sei hier für die LEDs eine Hauptabstrahlrichtung der LEDs im Sinne eines Lambert-Strahlers und für den Bildsensor die Hauptaufnahmerichtung, d.h. bei einem planaren CCD-Sensor oder CMOS-Sensor die Normale auf die Sensorfläche. Die erste LED, die zweite LED und der Bildsensor sind also sämtlich in die gleiche "Blickrichtung" ausgerichtet. Vorzugsweise ist die Blickrichtung distal von einer Stirnseite des Einführabschnitts gerichtet, kann allerdings zusätzlich oder alternativ lateral nach außen von einer Lateralseite des Einführabschnitts gerichtet sein. Bei der stirnseitigen Anordnung bedeutet "vorne" in Blickrichtung distal bezüglich des Instruments und bei der lateralen Anordnung bedeutet "vorne" in Blickrichtung lateral bezüglich des Instruments. Optional können also die erste LED, die zweite LED und der Bildsensor an einer gemeinsamen Wandung des Einführabschnitts angeordnet sein. Vorzugsweise ist dies eine distale Stirnseite des Einführabschnitts, wobei die Blickrichtung distal in Längsrichtung des Einführabschnitts verläuft. Insbesondere in dieser Ausführungsform ist der laterale Bauraum für die Platzierung der ersten LED, der zweiten LED und des Bildsensors an der Stirnseite sehr begrenzt. Es stehen in der Wandung des Einführabschnitts ggf. nur Ausnehmungen mit einem Durchmesser von 1 mm bis 1,5 mm pro LED bzw. Bildsensor zur Verfügung. In extremen Fällen kann der zur Verfügung stehende Durchmesser sogar nur 0,5 mm betragen.

Erfindungsgemäß ist die erste LED gegenüber der zweiten LED bezüglich der Blickrichtung nach vorne versetzt angeordnet. Da die erste LED für die Fluoreszenz-Endoskopie keinen vorgelagerten Lichtfilter braucht, kann die erste LED in Blickrichtung weiter nach vorne an einer Außenfläche des Einführabschnitts, vorzugsweise dessen Stirnfläche, angeordnet sein, wodurch sich ein Schlüssellocheffekt verringert und der ausgeleuchtete Raumwinkel vergrößert. Die zweite LED ist wegen des vorgelagerten Lichtfilters gegenüber der ersten LED bezüglich der Blickrichtung nach hinten versetzt. Das Lichtfilter muss zwar einerseits dick genug sein, um den zweiten Wellenlängenbereich stark genug ausfiltern zu können, damit die Weißlicht-Endoskopie mit dem Bildsensor ohne zusätzlichen Korrekturfilter im Bildpfad eine möglichst natürlich wirkende Echtfarb-Darstellung erzielt. Andererseits sollte das Lichtfilter so dünn wie möglich ausgestaltet sein, damit die zweite LED möglichst weit vorn platziert werden kann, sodass sich der Schlüssellocheffekt verringert und der ausgeleuchtete Raumwinkel vergrößert.

Optional kann das Lichtfilter ein Absorptionsfilter sein. Ein dichroitisches Filter kann zwar bei einer externen Lichtquelle mit einem Lichtleitsystem vorteilhaft sein, da es sich weniger stark aufheizt, ist aber zum Filtern vor einer LED im Einführabschnitt gegenüber einem Absorptionsfilter nachteilig, da ein dichroitisches Filter ein winkelabhängiges Transmissionsspektrum hat. Für ein Lichtleitsystem mit im Wesentlichen parallelen bzw. kollimierten Lichtbündeln ist dies wenig problematisch, führt aber bei einer LED mit einer Abstrahlcharakteristik eines Lambert-Strahlers zu unerwünschten Farbverzerrungen. Ein Absorptionsfilter weist eine solche Winkelabhängigkeit des Transmissionsspektrusm nur in geringem Maße auf, sodass unerwünschte Farbverzerrungen des LED-Lichts vernachlässigbar gering sind. Außerdem ist bei einer LED im Einführabschnitt die Lichtleistung gegenüber einer externen Lichtquelle wesentlich geringer, da Verluste im Lichtleitsystem nicht kompensiert werden müssen. Das Problem des Aufheizens des Absorptionsfilters und einer dadurch bedingten Beschädigung des Absorptionsfilters ist daher vor der zweiten LED nicht so stark ausgeprägt.

Optional kann das Lichtfilter ein Infrarot-Sperrfilter sein, wobei der erste Wellenlängenbereich im sichtbaren Lichtspektrum unterhalb von 550 nm liegt und der zweite Wellenlängenbereich im sichtbaren Lichtspektrum oberhalb von 550 nm. Ein erheblicher Teil des roten und infraroten Lichts der zweiten LED, das ohnehin im Vergleich zu einer anderen Lichtquelle als einer LED mit geringerer Intensität ausgestrahlt wird, wird vom Lichtfilter für die Weißlicht-Endoskopie ausgefiltert. Die Bildgebung ist einerseits natürlich und andererseits heizt das ausgefilterte langwellige Licht der zweiten LED nicht unnötig das Gewebe auf. Die Wärmentwicklung im Lichtfilter kann über eine Wandung des Einführabschnitts und/oder ein Kühlmittel abgeführt werden.

Optional kann der Bildsensor in einer zur Blickrichtung senkrechten Ebene im Wesentlichen den gleichen Abstand zur ersten LED haben wie zur zweiten LED, und vorzugsweise zwischen der ersten LED und der zweiten LED angeordnet sein. Dadurch kann ein Anwender einfach zwischen Weißlicht-Endoskopie und Fluoreszenz-Endoskopie wechseln, ohne dass sich der Beleuchtungswinkel und/oder die Beleuchtungsintensität bzw. der Schattenwurf im Bild stark ändern. Zwar könnte man durch unterschiedliche Ansteuerung der ersten LED und der zweiten LED unterschiedliche Abstände ggf. kompensieren, jedoch wäre dies energetisch weniger effizient. Vorzugsweise ist der Bildsensor zentral an der Stirnseite angeordnet. Die erste und zweite LED können seitlich versetzt davon mit möglichst geringem und gleichem lateralen Abstand an der Stirnseite angeordnet sein.

Optional können das erste Leuchtspektrum und das zweite Leuchtspektrum im Wesentlichen identisch oder unterschiedlich sein. Die Leuchtspektren der ersten und/oder zweiten LED können jeweils sowohl für die Fluoreszenz-Endoskopie als auch für die Weißlicht-Endoskopie geeignet sein, unabhängig davon, ob die jeweiligen Leuchtspektren im Wesentlichen identisch sind oder nicht. Die erste LED und die zweite LED können also vom gleichen Typ sein, d. h. baugleich sein, müssen es aber nicht. Es kann von Vorteil sein, dass die erste LED ein speziell für die Fluoreszenz-Endoskopie geeignetes erstes Leuchtspektrum aufweist. Beispielsweise kann die erste LED eine Blaulicht-emittierende LED zur Fluoreszenz-Anregung sein, während die zweite LED eine Weißlicht emittierende LED zur Weißlicht-Endoskopie sein kann. Das Leuchtspektrum der ersten und/oder zweiten LED kann oberhalb einer Wellenlänge von etwa 550 nm, d. h. im zweiten, roten Wellenlängenbereich, mit steigender Wellenlänge abfallen, sodass im Gegensatz zu einem Leuchtspektrum einer Halogen-, Xenon-, Halid- oder sonstigen Metall-Dampflampe die roten und infraroten Lichtanteile signifikant geringer sind und für die farbgetreue Weißlicht-Endoskopie entsprechend weniger stark ausgefiltert werden müssen.

Optional kann das Lichtfilter eine Lichteinlassseite und eine Lichtauslassseite aufweisen und zwischen der Lichteinlassseite und der Lichtauslassseite in Blickrichtung eine Dicke und orthogonal zur Blickrichtung einen Durchmesser haben, wobei die Dicke 0,3 mm bis 80% des Durchmessers beträgt. Die untere Grenze von 0,3 mm ergibt sich aus einem Mindestmaß, mit dem das Licht der zweiten LED im zweiten Wellenlängenbereich gefiltert werden muss, damit eine farbgetreue Bildgebung bei der Weißlicht-Endoskopie ohne Korrekturfilter im Bildpfad vor dem Bildsensor erzielt wird. Diese untere Grenze von 0,3 mm hängt vornehmlich von der maximalen optischen Dichte der verfügbaren Filtermaterialien ab. Die obere Grenze von 80% ergibt sich aus einem Höchstmaß, wie weit die zweite LED wegen des Lichtfilters in Blickrichtung zurückversetzt angeordnet sein kann, um mit einem noch tolerierbaren Schlüssellocheffekt einen Raumwinkel von mindestens 2,24 Steradiant ausreichend ausleuchten zu können. Ein Verhältnis von Durchmesser zu Dicke des Lichtfilters von etwa 1,5 kann besonders vorteilhaft sein. Optional kann das Lichtfilter eine Lichteinlassseite und eine Lichtauslassseite haben und zwischen der Lichteinlassseite und der Lichtauslassseite in Blickrichtung eine Dicke von 0,3 mm bis 1,2 mm haben. Beispielsweise bei einem Durchmesser von 1 mm kann das Lichtfilter etwa 0.67 mm dick sein oder bei einem Durchmesser von 1,5 mm etwa 1 mm dick.

Optional kann der Abstand der Lichteinlassseite des Lichtfilters von der Lichtabstrahlseite der zweiten LED weniger als 30% der Dicke des Lichtfilters in Blickrichtung betragen, idealerweise weniger als 10%. Dies ist vorteilhaft, um die zweite LED möglichst weit vorne in Blickrichtung anordnen zu können, um mit einem noch tolerierbaren Schlüssellocheffekt einen möglichst großen Raumwinkel ausreichend ausleuchten zu können. Ein minimaler Abstand ist jedoch gegenüber einem direkten Kontakt vorteilhaft, da das Licht beim Eintritt in das Lichtfilter von einem optisch weniger dichten Medium, d. h. mit kleinerem Brechungsindex wie etwa Luft, zum Einfallslot stark hin gebrochen und bei Austritt aus dem Lichtfilter vom Einfallslot entsprechend stark weg gebrochen wird, wodurch sich der Schlüssellocheffekt verringert. Bei direktem Kontakt wäre nämlich die Brechung bei Eintritt in das Lichtfilter zum Einfallslot hin wesentlich geringer, wodurch ein Anteil der schräg von der zweiten LED abgestrahlten Lichtstrahlen ggf. nicht mehr zur Lichtabstrahlseite des Lichtfilters hin gebrochen würde, sondern sich in der Zylindermantelfläche des Lichtfilters verlöre.

Optional kann das Lichtfilter und die zweite LED in einer Ausnehmung in einer Wandung des Einführabschnitts angeordnet sein, wobei die Wandung eine Außenfläche definiert und der Abstand einer Lichtabstrahlseite der zweiten LED von der Außenfläche höchstens zwei Drittel des Durchmessers der Ausnehmung beträgt. Die Außenfläche kann vorzugsweise eine Stirnfläche des Einführabschnitts sein. Die Ausnehmung, in der die zweite LED sitzt, bedingt einen gewissen "Tunnelblick" bzw. Schlüssellocheffekt, da die zweite LED wegen des vorgeschalteten Lichtfilters in Blickrichtung gegenüber der Außenfläche zurückversetzt angeordnet ist. Bei einem größeren Abstand als zwei Dritteln des Durchmessers führte der "Tunnelblick" bzw. Schlüssellocheffekt dazu, dass nur noch ein zu kleiner Raumwinkel ausreichend ausgeleuchtet werden könnte.

Optional können das zweite Leuchtspektrum der zweiten LED und das Transmissionsspektrum des Lichtfilters im zweiten Wellenlängenbereich zwischen 550 nm und 700 nm mit zunehmender Wellenlänge abfallen und die Transmission des Lichtfilters für von der zweiten LED ausgestrahltem Licht mit einer Wellenlänge von 600 nm 20% bis 45% betragen. Damit ergänzen sich die zweite LED und das Lichtfilter, den zweiten, roten Wellenlängenbereich bei möglichst geringer Dicke des Lichtfilters ausreichend auszufiltern, um eine farbgetreue Bildgebung für die Weißlicht-Endoskopie ohne zusätzlichen Korrekturfilter im Bildpfad vor dem Bildsensor zu erzielen.

Optional kann mindestens ein für Weißlicht durchlässiges Schutzelement in Blickrichtung vor einer Lichtabstrahlseite der ersten LED und/oder einer Lichtauslassseite des Lichtfilters angeordnet sein, wobei die Dicke des Schutzelements in Blickrichtung dünner als die Dicke des Lichtfilters in Blickrichtung ist. Das mindestens eine Schutzelement kann dabei ein möglichst dünnes Schutzglas, Schutzkunststoff und/oder eine auf der ersten LED bzw. dem Lichtfilter aufgebrachte Siliziumdioxidschicht sein. Das Schutzelement kann das Lichtfilter und/oder die erste LED gegen mechanische Beschädigung wie etwa Verkratzungen und chemische Beschädigung wie etwa durch aggressive Körperflüssigkeiten, Reinigungs- oder Aufbereitungsmedien und/oder Oxidation schützen.

Optional kann das mindestens eine Schutzelement in direktem Kontakt mit der Lichtabstrahlseite der ersten LED bzw. mit der Lichtauslassseite des Lichtfilters stehen oder der Abstand des mindestens einen Schutzelements von der Lichtabstrahlseite der ersten LED bzw. von der Lichtauslassseite des Lichtfilters weniger als 10% der Dicke des Lichtfilters in Blickrichtung betragen. Dies ist vorteilhaft, um die zweite LED möglichst weit vorne in Blickrichtung anordnen zu können, um mit einem noch tolerierbaren Schlüssellocheffekt einen möglichst großen Raumwinkel ausreichend ausleuchten zu können.

Optional können das Lichtfilter und/oder die zweite LED von einer spiegelnden Zylindermantelinnenfläche umgeben sein, die sich im Wesentlichen in Blickrichtung erstreckt. Die Zylindermantelinnenfläche kann beispielsweise von einer Ausnehmung in einer Wandung des Einführabschnitts gebildet sein. Alternativ oder zusätzlich dazu kann die spiegelnde Zylindermantelinnenfläche auf einer Zylinderaußenfläche des Lichtfilters und/oder der zweiten LED als eine radial nach innen spiegelnde Schicht aufgebracht sein.

Optional können eine Mehrzahl von $n \geq 2$ ersten LEDs und/oder eine Mehrzahl von $m \geq 2$ zweiten LEDs in einer zur Blickrichtung senkrechten Ebene n-zählig bzw. m-zählig rotationssymmetrisch bezüglich einer Blickrichtungsachse des Bildsensors im Einführabschnitt angeordnet sein. Dadurch wird sowohl für die Weißlichtendoskopie als auch für die Fluoreszenz-Endoskopie ein unerwünschter Schattenwurf reduziert. Es kann dabei eine gleiche Anzahl von ersten LEDs und zweiten LEDs, also n=m, vorgesehen sein, die in einem Kreis um den Bildsensor herum so angeordnet sind, das sich kreisumlaufend erste LEDs und zweite LEDs abwechseln. Wenn die zweiten LEDs als relativ lichtschwache blaue LEDs zur Fluoreszenz-Endoskopie verwendet werden, kann es allerdings beispielsweise vorteilhaft sein, mehr zweite LEDs vorzusehen als erste weiße LEDs, also m > n.

Die Offenbarung ist nachfolgend anhand von einem in den Zeichnungen dargestellten Ausführungsbeispiel näher erläutert. Es zeigen:

Fig. 1 einen schematischen Längsschnitt durch einen distalen Abschnitt eines Einführabschnitts gemäß einem Ausführungsbeispiels des hierin offenbarten medizinisch-endoskopischen Instruments;

Fig. 2 und 3 Vorderansichten auf eine Stirnseite eines Einführabschnitts gemäß zweier Ausführungsbeispiele des hierin offenbarten medizinisch-endoskopischen Instruments;

Fig. 4 und 5 Vorderansichten auf eine Stirnseite eines Einführabschnitts gemäß zweier weiterer Ausführungsbeispiele des hierin offenbarten medizinisch-endoskopischen Instruments;

Fig. 6 und 7 Leuchtspektren der ersten und zweiten LED gemäß einem Ausführungsbeispiel des hierin offenbarten medizinisch-endoskopischen Instruments; und

Fig. 8 bis 11 verschiedene beispielhafte Transmissionsspektren des Lichtfilters gemäß Ausführungsbeispielen des hierin offenbarten medizinisch-endoskopischen Instruments.

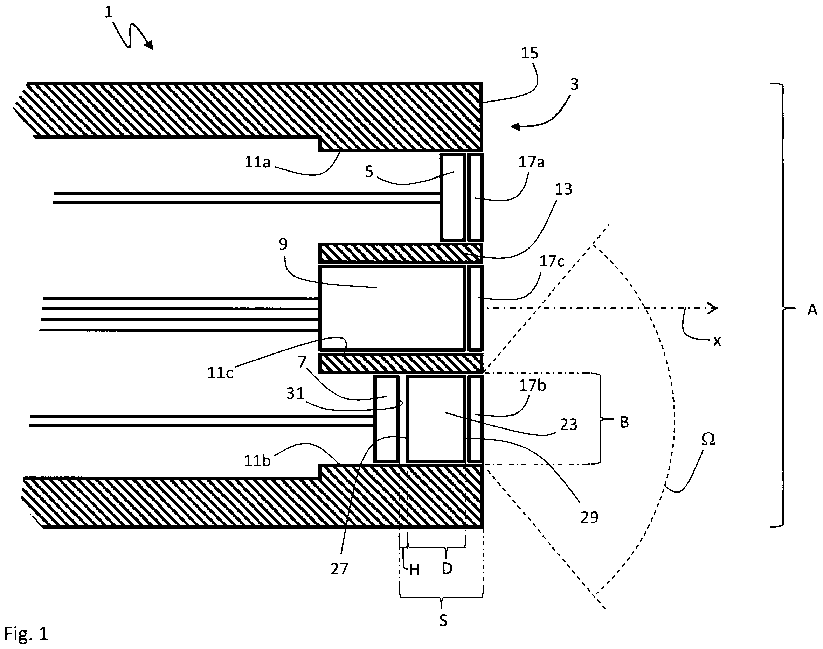

Fig. 1 zeigt einen distalen Endabschnitt eines Einführabschnitts 1 eines medizinisch-endoskopisches Instruments. Der Einführabschnitt 1 ist dazu vorgesehen, minimal-invasiv in einen menschlichen oder tierischen Körper eingeführt zu werden, um diesen mit Licht ausleuchten bzw. bestrahlen zu können und eine Video- bzw. Bildübertragung aus dem Inneren des Körpers zu ermöglichen. Um das Einführen minimal-invasiv zu gestalten, ist ein Außendurchmesser A des Einführabschnitts 1 möglichst gering und beträgt in diesem Ausführungsbeispiel weniger als 5 mm.

An einer distalen Stirnseite 3 des Einführabschnitts 1 sind nebeneinander eine erste LED 5, eine zweite LED 7 und ein Bildsensor 9 angeordnet, welche in eine gemeinsame Blickrichtung x ausgerichtet sind, die in diesem Ausführungsbeispiel der Längsrichtung des Einführabschnitts 1 entspricht. Die erste LED 5, die zweite LED 7 und der Bildsensor 9 sind jeweils in einer Ausnehmung 11a,b,c in einer Stirnwandung 13 des Einführabschnitts 1 angeordnet. Die Stirnwandung 13 definiert eine Außenfläche 15 an der Stirnseite 3 des Einführabschnitts 1. Die erste LED 5, die zweite LED 7 und der Bildsensor 9 sind jeweils hinter Schutzelementen 17a,b,c in Form von dünnen Schutzglasscheiben angeordnet, die sämtlich mit der Außenfläche 15 an der Stirnseite 3 des Einführabschnitts 1 fluchten und gegen mechanische Beschädigung wie etwa Verkratzungen und chemische Beschädigung wie etwa durch aggressive Körperflüssigkeiten, Reinigungs- oder Aufbereitungsmedien und/oder Oxidation schützen. Die Schutzelemente 17a,b,c können auch als eine gemeinsame die erste LED 5, die zweite LED 7 und den Bildsensor 9 übergreifende Schutzglasscheibe ausgestaltet sein. Die Schutzelemente 17a,b,c sind für Weißlicht durchlässig und haben in diesem Ausführungsbeispiel einen Brechungsindex von mindestens 1,75 sowie eine höhere Bruchfestigkeit und Härte als herkömmliches optisches Glas. Die Schutzelemente 17a,b,c können aus einem synthetischen monokristallinen Kristall ausgebildet sein.

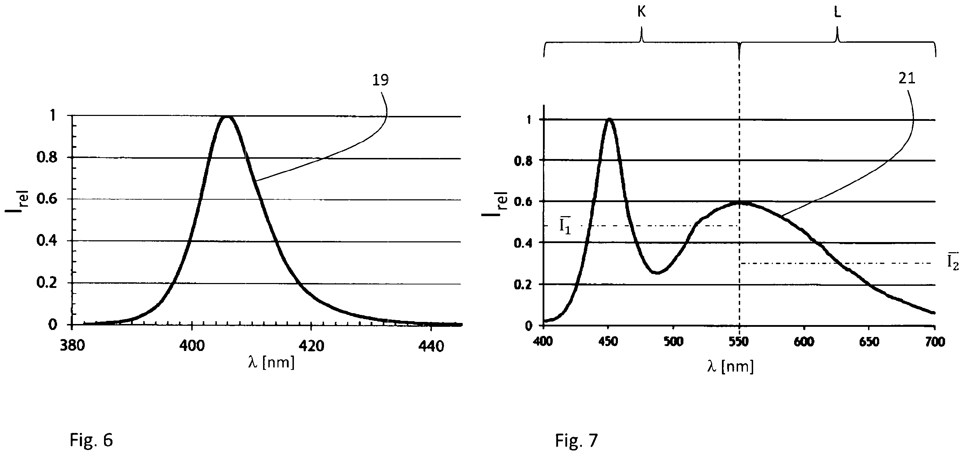

Die erste LED 5 weist ein für eine Fluoreszenz-Endoskopie geeignetes erstes Leuchtspektrum 19 (siehe Fig. 6) auf, das hier einen Peak bei 406 nm mit einer Halbwertsbreite von 12 nm im blauen Wellenlängenbereich hat. Mit diesem blauen Licht der ersten LED 5 kann im Rahmen einer photodynamischen Diagnose (PDD) und/oder photodynamischen Therapie (PDT) ein Photosensibilisator, der sich selektiv an pathologischem Gewebe anreichert, zum Fluoreszieren im roten Wellenlängenbereich gebracht werden. Solch ein Fluoreszieren im roten Wellenlängenbereich kann durch den Bildsensor 9, dem kein Korrekturfilter vorgeschaltet ist, gut aufgenommen werden. Dem Bildsensor 9 kann ein Objektiv und/oder ein Langpassfilter vorgeschaltet sein (nicht in Fig. 1 gezeigt). Solch ein Langpassfilter stellt allerdings keinen Korrekturfilter zum Angleichen der spektralen Sensorempfindlichkeit an die spektrale Empfindlichkeit des menschlichen Auges dar, sondern blockt lediglich direkt vom Körper zurückgestreutes kurzwelliges blaues Licht der ersten LED 5.

Die zweite LED 7 weist ein für eine Weißlicht-Endoskopie geeignetes zweites Leuchtspektrum 21 (siehe Fig. 7) auf, das hier in einem ersten Wellenlängenbereich K von 400 nm bis 550 nm einen Peak aufweist und in einem zweiten Wellenlängenbereich L von 550 nm bis 700 nm mit steigender Wellenlänge abfällt. Die erste LED 5 kann alternativ zu dem in Fig. 6 dargestellten ersten Leuchtspektrum 19 das gleiche Leuchtspektrum 21 wie die zweite LED 7 aufweisen, sofern sich damit die für die vorgesehene Fluoreszenz-Endoskopie erforderliche Fluoreszenzanregung bewirken lässt. In diesem Fall können die erste LED 5 und die zweite LED 7 vom gleichen Typ sein.

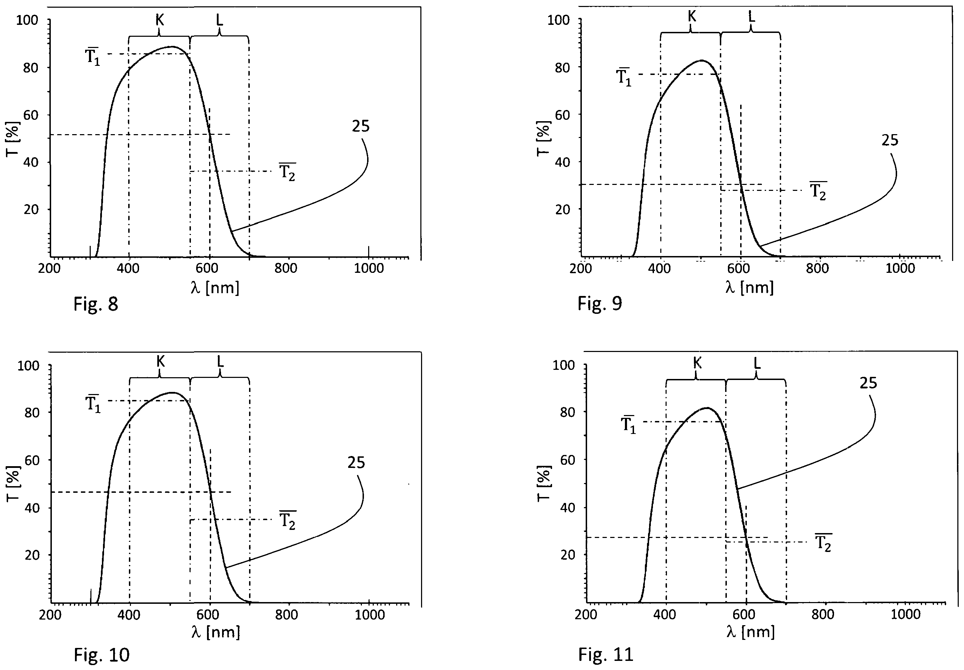

Die zweite LED 7 ist in diesem Ausführungsbeispiel gegenüber der ersten LED 5 bezüglich der Blickrichtung x nach hinten versetzt angeordnet. Dies liegt daran, dass vor der zweiten LED 7 und hinter dem Schutzelement 17b ein Infrarot-Sperrfilter 23 angeordnet ist, das ein Transmissionsspektrum 25 gemäß einer der Figuren 8 bis 11 aufweist. Das Licht der zweiten LED 7, das gemäß dem zweiten Leuchtspektrum 21 (siehe Figur 7) im unteren ersten Wellenlängenbereich K im Mittel deutlich intensiver strahlt als im oberen zweiten Wellenlängenbereich L wird vom Infrarot-Sperrfilter 23 gemäß dem Transmissionsspektrum 25 (siehe Figuren 8 bis 11) im oberen zweiten Wellenlängenbereich L im Mittel weniger durchgelassen als im ersten Wellenlängenbereich K. Das zweite Leuchtspektrum 21 der zweiten LED 7 und das Transmissionsspektrum 25 des Infrarot-Sperrfilters 23 ergänzen sich also bei der Reduzierung des Lichts im oberen zweiten Wellenlängenbereich L. Dies hat den positiven Effekt, dass die Dicke D des Infrarot-Sperrfilters 23 zwischen einer Lichteinlassseite 27 des Infrarot-Sperrfilters 23 und einer Lichtauslassseite 29 des Infrarot-Sperrfilters 23 dünner ausgestaltet werden kann, damit der Abstand S von der Lichtauslassseite 29 der zweiten LED 7 zur Außenfläche 15, um den die zweite LED 7 wegen des Infrarot-Sperrfilters 23 nach hinten versetzt sein muss, möglichst gering ausfällt.

Die Ausnehmung 11b in einer Stirnwandung 13 des Einführabschnitts 1, in der die zweite LED 7, das Infrarot-Sperrfilter 23 und das Schutzelement 17b passgenau eingelassen sind, hat hier einen Durchmesser B. Die Ausnehmung 11b kann mit einer spiegelnden Zylindermantelinnenfläche versehen sein, muss es aber nicht, da der Schlüssellocheffekt durch andere Maßnahmen in den hier beschriebenen Ausführungsformen bereits hinreichend reduziert werden kann. Alternativ zur Stirnwandung 13 könnte eine Hülse mit einem Innendurchmesser B die zweite LED 7, das Infrarot-Sperrfilter 23 und das Schutzelement 17b passgenau und zusätzlich ggf. spiegelnd einfassen. Die radiale Außenfläche des Infrarot-Sperrfilters 23 könnte ggf. mit einer spiegelnden Metallschicht versehen sein.

Das Verhältnis B/S vom Durchmesser B und dem Abstand S zwischen der Lichtauslassseite 29 der zweiten LED 7 und der Außenfläche 15, um den die zweite LED 7 wegen des Infrarot-Sperrfilters 23 nach hinten versetzt ist, bestimmt einen Raumwinkel $\Omega$, der durch die zweite LED 7 ausgeleuchtet wird. Der ausgeleuchtete Raumwinkel $\Omega$ sollte mindestens 2,24 Steradiant betragen, d.h. etwa 35% einer Einheitshalbkugelfläche, um einen Schlüssellocheffekt nach Möglichkeit zu reduzieren. Das Verhältnis B/S beträgt hier in etwa 1,5.

Um den Abstand S möglichst kurz zu gestalten, wird also zum einen eine möglichst geringe Dicke D des Infrarot-Sperrfilters 23 gewählt, z. B. 0,3 mm bis 80% des Durchmessers B, welche eine noch ausreichend hohe Filterwirkung bietet. Zum anderen liegt zwischen einer Lichtabstrahlseite 31 der zweiten LED 7 und der Lichteinlassseite 27 des Infrarot-Sperrfilters 23 ein minimaler Abstand H von weniger als 10% der Dicke D des Infrarot-Sperrfilters 23, um eine Brechung hin zum Einfallslot beim Eintritt in den Infrarot-Sperrfilter 23 dazu zu nutzen, um den Schlüssellocheffekt zu verringern.

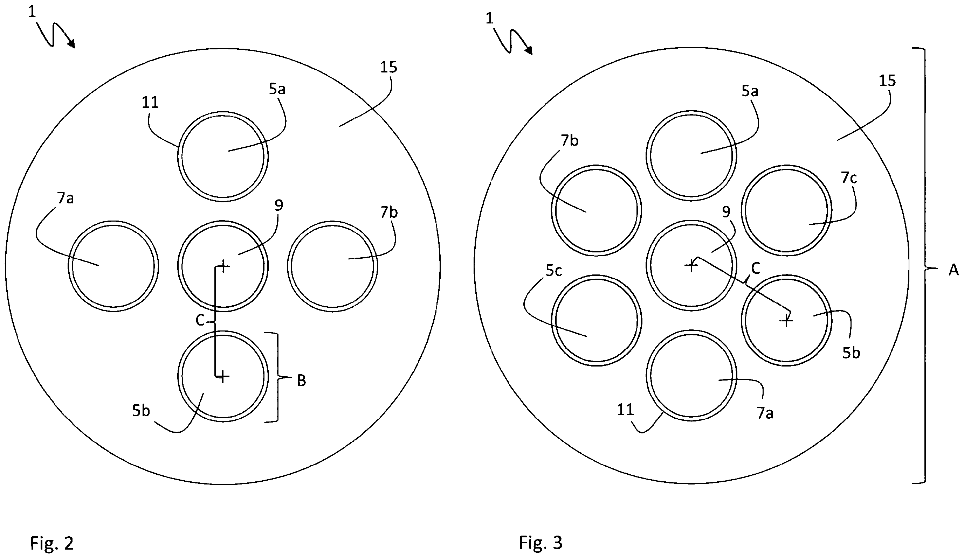

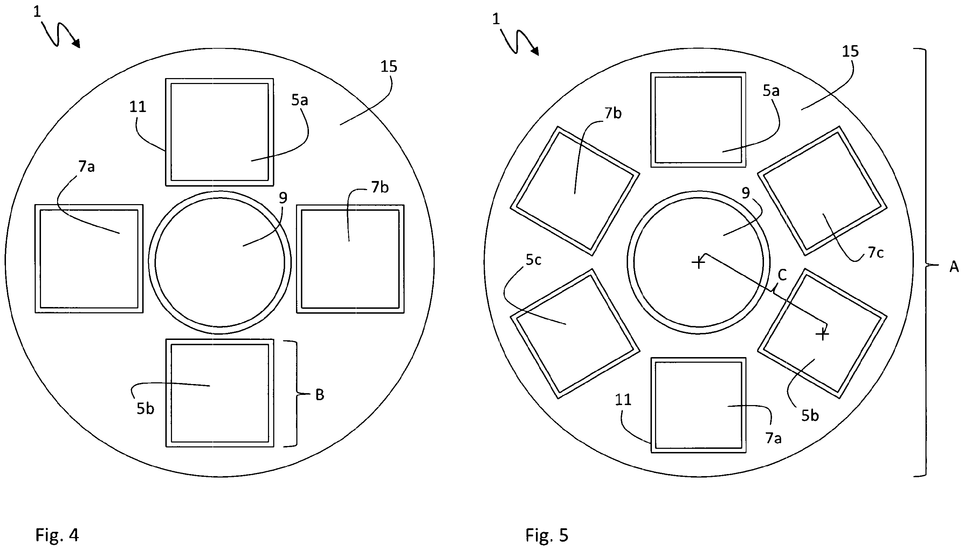

In den Vorderansichten auf die Stirnseite 3 des Einführabschnitts 1 gemäß der Figuren 2 bis 5 sind verschiedene Ausführungsbeispiele mit einer Mehrzahl von ersten LEDs 5a-c bzw. zweiten LEDs 7a-c gezeigt. Koaxial mittig in der Stirnwandung 13 des Einführabschnitts 1 ist jeweils der Bildsensor 9 angeordnet. Um den Bildsensor 9 herum sind jeweils eine Mehrzahl von $n \geq 2$ ersten LEDs 5a-c und eine Mehrzahl von $m \geq 2$ zweiten LEDs 7a-c in einer zur Blickrichtung x senkrechten Ebene n-zählig bzw. m-zählig rotationssymmetrisch bezüglich einer Blickrichtungsachse x des Bildsensors im Einführabschnitt 1 angeordnet. In Figuren 2 und 4 ist n=2 und m=2, wobei die ersten LEDs 5a,b jeweils diametral einander gegenüberliegend den gleichen Abstand C zum zentralen Bildsensor 9 haben wie die ebenfalls jeweils diametral einander gegenüberliegenden zweiten LEDs 7a,b. In Figuren 3 und 5 ist n=3 und m=3, wobei die ersten LEDs 5a-c jeweils 120° umfänglich versetzt zueinander liegend den gleichen Abstand C zum zentralen Bildsensor 9 haben wie die ebenfalls jeweils 120° umfänglich versetzt zueinander liegenden zweiten LEDs 7a,b. In den Ausführungsbeispielen der Figuren 4 und 5 haben die Ausnehmungen 11 im Gegensatz zu den Ausführungsbeispielen der Figuren 2 und 3 keinen kreisförmigen, sondern einen rechteckigen bzw. quadratischen Querschnitt. Im Falle einer rechteckigen Ausnehmung 11 entspricht der Durchmesser B der Länge der kürzeren Rechteckseite.

Das erste Leuchtspektrum 19 der ersten LED 5 und das zweite Leuchtspektrum 21 der zweiten LED 7 sind beispielhaft in den Figuren 6 und 7 gezeigt, wobei alternativ das erste Leuchtspektrum 19 der ersten LED 5 gleich sein kann wie das zweite Leuchtspektrum 21 der zweiten LED 7. Die erste LED 5 ist hier eine blaue LED mit dem ersten Leuchtspektrum 19, das für eine Fluoreszenz-Endoskopie geeignet ist und einen Peak bei 406 nm mit einer Halbwertsbreite von 12 nm im blauen Wellenlängenbereich hat. Die zweite LED 5 ist hier eine weiß leuchtende LED mit dem zweiten Leuchtspektrum 21, das hier im ersten Wellenlängenbereich K von 400 nm bis 550 nm einen Peak aufweist und im zweiten Wellenlängenbereich L von 550 nm bis 700 nm mit steigender Wellenlänge abfällt. Im Mittel leuchtet die zweite LED 5 daher im ersten Wellenlängenbereich K intensiver als im zweiten Wellenlängenbereich L. In den Figuren 5 und 7 ist eine relative Intensität $I_{rel}$ dimensionlos über die Wellenlänge $\lambda$ in nm abgetragen. Die relative Intensität $I_{rel}$ ist so definiert, dass sie 1 beträgt beim Intensitätsmaximum. Die mittlere Intensität in einem Wellenlängenbereich zwischen einer Wellenlänge $\lambda_1$ und einer Wellenlänge $\lambda_2$ ist hier definiert als $\overline{I} = \int_{\lambda_1}^{\lambda_2} \frac{I(\lambda)}{\lambda_2 - \lambda_1} d\lambda$. Erstreckt sich also der erste Wellenlängenbereich K von einer Wellenlänge $\lambda_1 = 400$ nm zu einer Wellenlänge $\lambda_2 = 550$ nm, und der zweite Wellenlängenbereich L von einer Wellenlänge $\lambda_3 = 550$ nm zu einer Wellenlänge $\lambda_4 = 700$ nm, so ergibt sich hier

$$\overline{I_1} = \int_{\lambda_1}^{\lambda_2} \frac{I_1(\lambda)}{\lambda_2 - \lambda_1} d\lambda = 0,5 > \overline{I_2} = \int_{\lambda_3}^{\lambda_4} \frac{I_2(\lambda)}{\lambda_4 - \lambda_3} d\lambda = 0,3$$
.

Die in den Figuren 8 bis 11 beispielhaft gezeigten Transmissionsspektren 25 des Infrarot-Sperrfilters 23 entsprechen unterschiedlichen Filtertypen und -dicken. In Fig. 8 ist das Transmissionsspektrum 25 eines Bandfilters BG 39 (Blauglass) der Firma Schott mit einer Dicke von 1 mm gezeigt. In Fig. 10 ist das gleiche Infrarot-Sperrfilter 23 mit einer von Dicke von 1,2 mm gezeigt. Man erkennt, dass das Transmissionsspektrum 25 mit der Dicke D des Infrarot-Sperrfilters 23 skaliert. Beispielsweise beträgt die Transmission bei 600 nm etwa 51% bei 1 mm Dicke und nur noch 46% bei 1,2 mm Dicke. Eine stärkere Filterwirkung im oberen Wellenlängenbereich L kann mit einem anderen Filterglass erzielt werden. In Fig. 9 ist das Transmissionsspektrum 25 eines Bandfilters BG 62 (Blauglass) der Firma Schott mit einer Dicke von 1 mm gezeigt. Hier beträgt die Transmission bei 600 nm nur etwa 28% bei 1 mm Dicke. Um die Dicke D weiter reduzieren zu können, kann auch das Bandfilter BG 67 (Blauglass) der Firma Schott gewählt werden, dessen Transmissionsspektrum 25 in Fig. 11 für eine Dicke von 0,67 mm gezeigt ist. Die Transmission bei 600 nm liegt hier ebenfalls unter 30%.

Durch die Möglichkeit von Fluoreszenzlicht mit der ersten LED 5 und mittels des Lichtfilters 23 gefiltertem Weißlicht mit der zweiten LED 7 kann mit nur dem einen Bildsensor 9 einfach und schnell zwischen Fluoreszenz-Endoskopie und Weißlicht-Endoskopie umgeschaltet werden. Der Bildsensor 9 kommt dabei ohne einen Korrekturfilter in Form von einem Kurzpassfilter aus und ist dadurch insbesondere bei der Weißlicht-Endoskopie effizienter, da Gewebe erwärmendes langwelliges Licht, für das der Bildsensor 9 ohnehin für die Weißlicht-Endoskopie zu empfindlich ist, mittels der Kombination von bereits im oberen Wellenlängenbereich L schwächerem Licht der zweiten LED 7 und dem Infrarot-Sperrfilter 23 gar nicht erst vom Instrument ausgestrahlt wird. Ein Schlüssellocheffekt kann durch eine geeignete Wahl des Infrarot-Sperrfilters 23 mit geeigneter Dicke bei für minimalinvasive Eingriffe beschränktem Durchmesser B reduziert werden.

Die nummerierten Bezeichnungen der Bauteile oder Bewegungsrichtungen als "erste", "zweite", "dritte" usw. sind hierin rein willkürlich zur Unterscheidung der Bauteile oder Bewegungsrichtungen untereinander gewählt und können beliebig anders gewählt werden. Es ist damit kein Bedeutungsrang verbunden. Eine Bezeichnung eines Bauteils oder technischen Merkmals als "erstes" soll nicht dahingehend missverstanden werden, dass es ein zweites Bauteil oder technisches Merkmal dieser Art geben muss. Außerdem können etwaige Verfahrensschritte, soweit nicht explizit anders erläutert oder zwingend erforderlich, in beliebiger Reihenfolge und/oder zeitlich teilweise oder ganz überlappend durchgeführt werden.

## Patentansprüche

1. Medizinisch-endoskopisches Instrument mit einem distalen länglichen Einführabschnitt (1) zum minimal-invasiven Einführen in einen menschlichen oder tierischen Körper, wobei der Einführabschnitt (1) mindestens eine erste LED (5), eine zweite LED (7) und einen Bildsensor (9) aufweist, wobei die erste LED (5), die zweite LED (7) und der Bildsensor (9) in eine gemeinsame Blickrichtung (x) ausgerichtet sind,
wobei
die erste LED (5) ein für eine Fluoreszenz-Endoskopie geeignetes erstes Leuchtspektrum (19) und die zweite LED (7) ein für eine Weißlicht-Endoskopie geeignetes zweites Leuchtspektrum (21) aufweist, wobei ein Lichtfilter (23) in Blickrichtung (x) vor der zweiten LED (7) angeordnet ist, wobei das Lichtfilter (23) ein Transmissionsspektrum (25) aufweist, wobei die zweite LED (7) dazu ausgestaltet ist, gemäß dem zweiten Leuchtspektrum (21) in einem ersten Wellenlängenbereich (K) im Mittel intensiver zu strahlen als in einem zweiten Wellenlängenbereich (L), und wobei das Lichtfilter (23) dazu ausgestaltet ist, von der zweiten LED (7) ausgestrahltes Licht gemäß dem Transmissionsspektrum (25) im zweiten Wellenlängenbereich (L) im Mittel weniger durchzulassen als im ersten Wellenlängenbereich (K), **dadurch gekennzeichnet, dass** die erste LED (5) gegenüber der zweiten LED (7) bezüglich der Blickrichtung (x) nach vorne versetzt angeordnet ist.

2. Medizinisch-endoskopisches Instrument nach Anspruch 1, wobei die erste LED (5), die zweite LED (7) und der Bildsensor (9) an einer gemeinsamen Wandung (13) des Einführabschnitts (1) angeordnet sind.

3. Medizinisch-endoskopisches Instrument nach Anspruch 1 oder 2, wobei die erste LED (5), die zweite LED (7) und der Bildsensor (9) an einer distalen Stirnseite (3) des Einführabschnitts (1) angeordnet sind und die Blickrichtung (x) distal in Längsrichtung des Einführabschnitts (1) verläuft.

4. Medizinisch-endoskopisches Instrument nach einem der vorhergehenden Ansprüche, wobei das Lichtfilter (23) ein Absorptionsfilter ist.

5. Medizinisch-endoskopisches Instrument nach einem der vorhergehenden Ansprüche, wobei das Lichtfilter (23) ein Infrarot-Sperrfilter ist, wobei der erste Wellenlängenbereich (K) im sichtbaren Lichtspektrum unterhalb von 550 nm liegt und der zweite Wellenlängenbereich (L) im sichtbaren Lichtspektrum oberhalb von 550 nm.

6. Medizinisch-endoskopisches Instrument nach einem der vorhergehenden Ansprüche, wobei der Bildsensor (9) in einer zur Blickrichtung (x) senkrechten Ebene im Wesentlichen den gleichen Abstand (C) zur ersten LED (5) hat wie zur zweiten LED (7) und vorzugsweise zwischen der ersten LED (5) und der zweiten LED (7) angeordnet ist.

7. Medizinisch-endoskopisches Instrument nach einem der vorhergehenden Ansprüche, wobei das erste Leuchtspektrum (19) und das zweite Leuchtspektrum (21) unterschiedlich oder im Wesentlichen identisch sind.

8. Medizinisch-endoskopisches Instrument nach einem der vorhergehenden Ansprüche, wobei die erste LED (5) eine Weißlicht emittierende LED und die zweite LED (7) eine Blaulicht emittierende LED ist, oder die erste LED (5) und die zweite LED (7) vom gleichen Typ sind.

9. Medizinisch-endoskopisches Instrument nach einem der vorhergehenden Ansprüche, wobei das Lichtfilter (23) eine Lichteinlassseite (27) und eine Lichtauslassseite (29) aufweist und zwischen der Lichteinlassseite (27) und der Lichtauslassseite (29) in Blickrichtung (x) eine Dicke (D) und orthogonal zur Blickrichtung (x) einen Durchmesser (B) hat, wobei die Dicke 0,3 mm bis 80% des Durchmessers (B) beträgt.

10. Medizinisch-endoskopisches Instrument nach einem der vorhergehenden Ansprüche, wobei das Lichtfilter (23) eine Lichteinlassseite (27) und eine Lichtauslassseite (29) hat und zwischen der Lichteinlassseite (27) und der Lichtauslassseite (29) in Blickrichtung (x) eine Dicke (D) von 0,3 mm bis 1,2 mm hat.

11. Medizinisch-endoskopisches Instrument nach einem der vorhergehenden Ansprüche, wobei der Abstand (H) der Lichteinlassseite (31) des Lichtfilters (23) von der Lichtabstrahlseite (31) der zweiten LED (7) weniger als 30%, vorzugsweise weniger als 10%, der Dicke (D) des Lichtfilters (23) in Blickrichtung (x) beträgt.

12. Medizinisch-endoskopisches Instrument nach einem der vorhergehenden Ansprüche, wobei das Lichtfilter (23) und die zweite LED (7) in einer Ausnehmung (11b) in einer Wandung (13) des Einführabschnitts (1) angeordnet sind, wobei die Wandung (13) eine Außenfläche (15) definiert und der Abstand (S) einer Lichtabstrahlseite (31) der zweiten LED (7) von der Außenfläche (15) höchstens zwei Drittel des Durchmessers (B) der Ausnehmung (11b) beträgt.

13. Medizinisch-endoskopisches Instrument nach einem der vorhergehenden Ansprüche, wobei das zweite Leuchtspektrum (21) der zweiten LED (7) und das Transmissionsspektrum (25) des Lichtfilters (23) im zweiten Wellenlängenbereich (L) zwischen 550 nm und 700 nm mit zunehmender Wellenlänge abfallen und die Transmission des Lichtfilters (23) für von der zweiten LED (7) ausgestrahltem Licht mit einer Wellenlänge von 600 nm 20% bis 45% beträgt.

14. Medizinisch-endoskopisches Instrument nach einem der vorhergehenden Ansprüche, wobei mindestens ein für Weißlicht durchlässiges Schutzelement (17a,b) in Blickrichtung (x) vor einer Lichtabstrahlseite der ersten LED (5) und/oder einer Lichtauslassseite (29) des Lichtfilters (23) angeordnet ist, wobei die Dicke des Schutzelements (17a,b) in Blickrichtung (x) dünner als die Dicke (D) des Lichtfilters (23) in Blickrichtung (x) ist.

15. Medizinisch-endoskopisches Instrument nach Anspruch 14, wobei das mindestens eine Schutzelement (17a,b) in direktem Kontakt mit der Lichtabstrahlseite der ersten LED (5) bzw. mit der Lichtauslassseite (29) des Lichtfilters (23) steht oder ein Abstand des mindestens einen Schutzelements (17a,b) von der Lichtabstrahlseite der ersten LED (5) bzw. von der Lichtauslassseite (29) des Lichtfilters (23) weniger als 30%, vorzugsweise weniger als 10%, der Dicke (D) des Lichtfilters in Blickrichtung (x) beträgt.

16. Medizinisch-endoskopisches Instrument nach Anspruch 15, wobei zwischen dem mindestens einen Schutzelement (17a,b) und der Lichtabstrahlseite der ersten LED (5) bzw. der Lichtauslassseite (29) des Lichtfilters (23) ein Medium angeordnet ist, das einen Brechungsindex aufweist, der im Bereich von 90% bis 110% des Brechungsindexes des Lichtfilters (23) liegt.

17. Medizinisch-endoskopisches Instrument nach 15 oder 16, wobei das mindestens eine Schutzelement (17a,b) ein Schutzglas, ein Schutzkunststoff und/oder eine auf der ersten LED bzw. dem Lichtfilter (23) aufgebrachte Siliziumdioxidschicht ist.

18. Medizinisch-endoskopisches Instrument nach einem der vorhergehenden Ansprüche, wobei das Lichtfilter (23) und/oder die zweite LED (7) von einer spiegelnden Zylindermantelinnenfläche umgeben sind, die sich im Wesentlichen in Blickrichtung (x) erstreckt.

19. Medizinisch-endoskopisches Instrument nach Anspruch 18, wobei die spiegelnde Zylindermantelinnenfläche von einer Ausnehmung (11a,b) in einer Wandung (13) des Einführabschnitts (1) gebildet ist.

20. Medizinisch-endoskopisches Instrument nach einem der vorhergehenden Ansprüche, wobei eine Mehrzahl von n $\geq$ 2 ersten LEDs (5a-c) und/oder eine Mehrzahl von m > 2 zweiten LEDs (7a-c) in einer zur Blickrichtung (x) senkrechten Ebene n-zählig bzw. m-zählig rotationssymmetrisch bezüglich einer Blickrichtungsachse (x) des Bildsensors (9) im Einführabschnitt (1) angeordnet sind.

**Claims**

1. A medical-endoscopic instrument with a distal elongate insertion section (1) for the minimal-invasive introduction into a human or animal body, wherein the insertion section (1) comprises at least one first LED (5), a second LED (7) and a picture sensor (9), wherein the first LED (5), the second LED (7) and the picture sensor (9) are aligned in a common viewing direction (x), wherein the first LED (5) comprises a first light spectrum (19) which is suitable for fluorescence endoscopy and the second LED (7) comprises a second light spectrum (21) which is suitable for white light endoscopy, wherein a light filter (23) is arranged in front of the second LED (7) in the viewing direction (x), wherein the light filter (23) has a transmission spectrum (25), wherein the second LED (7) is designed to irradiate according to the second light spectrum (21) on average more intensively in a first wavelength region (K) than in a second wavelength region (L), and wherein the light filter (23) is designed to let through light which is emitted by the second LED (7), according to the transmission spectrum (25), on average less in the second wavelength region (L) than in the first wavelength region (K), **characterized in that** the first LED (5) is arranged offset to the front relative to the second LED (7) with respect to the viewing direction (x).

2. A medical-endoscopic instrument according to claim 1, wherein the first LED (5), the second LED (7) and the picture sensor (9) are arranged on a common wall (13) of the insertion section (1).

3. A medical-endoscopic instrument according to claim 1 or 2, wherein the first LED (5), the second LED (7) and the picture sensor (9) are arranged on a distal face side (3) of the insertion section (1) and the viewing direction (x) runs distally in the longitudinal direction of the insertion section (1).

4. A medical-endoscopic instrument according to one of the preceding claims, wherein the light filter (23) is an absorption filter.

5. A medical-endoscopic instrument according to one of the preceding claims, wherein the light filter (23) is an infrared blocking filter, wherein the first wavelength region (K) lies in the visible light spectrum below 550 nm and the second wavelength region (L) in the visible light spectrum above 550 nm.

6. A medical-endoscopic instrument according to one of the preceding claims, wherein the picture sensor (9) in a plane which is perpendicular to the viewing direction (x) has essentially the same distance (C) to the first LED (5) as to the second LED (7) and preferably is arranged between the first LED (5) and the second LED (7).

7. A medical-endoscopic instrument according to one of the preceding claims, wherein the first light spectrum (19) and the second light spectrum (21) are different or essentially identical.

8. A medical-endoscopic instrument according to one of the preceding claims, wherein the first LED (5) is an LED which emits white light and the second LED (7) is an LED which emits blue light, or the first LED (5) and the second LED (7) are of the same type.

9. A medical-endoscopic instrument according to one of the preceding claims, wherein the light filter (23) comprises a light inlet side (27) and a light outlet side (29) and between the light inlet side (27) and light outlet side (29) in the viewing direction (x) has a thickness (D) and orthogonally to the viewing direction (x) a diameter (B), wherein the thickness is 0.3 mm up to 80% of the diameter.

10. A medical-endoscopic instrument according to one of the preceding claims, wherein the light filter (23) has a light inlet side (27) and a light outlet side (29) and a thickness (D) of 0.3 mm to 1.2 mm between the light inlet side (27) and the light outlet side (29) in the viewing direction (x).

11. A medical-endoscopic instrument according to one of the preceding claims, wherein the distance (H) of the light inlet side (31) of the light filter (23) to the light irradiation side (31) of the second LED (7) is less than 30%, preferably less than 10% of the thickness (D) of the light filter (23) in the viewing direction (x).

12. A medical-endoscopic instrument according to one of the preceding claims, wherein the light filter (23) and the second LED (7) are arranged in a recess (11b) in a wall (13) of the insertion section (1), wherein the wall (13) defines an outer surface (15) and the distance (S) of a light irradiation side (31) of the second LED (7) to the outer surface (15) at the most is two thirds of the diameter (B) of the recess (11b).

13. A medical-endoscopic instrument according to one of the preceding claims, wherein the second light spectrum (21) of the second LED (7) and the transmission spectrum (25) of the light filter (23) in the second wavelength region (L) between 550 nm and 700 nm decreases with an increasing wavelength and the transmission of the light filter (23) for light emitted by the second LED (7) with a wavelength of 600 nm is 20% to 45%.

14. A medical-endoscopic instrument according to one of the preceding claims, wherein at least one protective element (17a,b) which is transparent to white light is arranged in front of a light irradiation side of the first LED (5) and/or of a light outlet side (29) of the light filter (23) in the viewing direction (x), wherein the thickness of the protective element (17a,b) in the viewing direction (x) is thinner than the thickness (D) of the light filter (23) in the viewing direction (x).

15. A medical-endoscopic instrument according to claim 14, wherein the at least one protective element (17a, b) is in direct contact with the light irradiation side of the first LED (5) or with the light outlet side (29) of the light filter (23) or a distance of the at least one protective element (17a,b) to the light irradiation side of the first LED (5) or to the light outlet side (29) of the light filter (23) is less than 30%, preferably less than 10% of the thickness (D) of the light filter in the viewing direction (x).

16. A medical-endoscopic instrument according to claim 15, wherein a medium which has a refractive index which lies in the region of 90% to 110% of the refractive index of the light filter (23) is arranged between the at least one protective element (17a,b) and the light irradiation side of the first LED (5) or the light outlet side (29) of the light filter (23).

17. A medical-endoscopic instrument according to claim 15 or 16, wherein least one protective element (17a, b) is a protective glass, a protective plastic and/or a silicon dioxide layer which is deposited on the first LED or on the light filter (23).

18. A medical-endoscopic instrument according to one of the preceding claims, wherein the light filter (23) and/or the second LED (7) are surrounded by a mirroring cylinder lateral inner surface which extends essentially in the viewing direction (x).

19. A medical-endoscopic instrument according to claim 18, wherein the mirroring cylindrical lateral inner surface is formed for example by a recess (11a, b) in a wall (13) of the insertion section (1).

20. A medical-endoscopic instrument according to one of the preceding claims, wherein a plurality of n ≥ 2 first LEDs (5a-c) and/or a plurality of m ≥ 2 second LEDs (7a-c) are arranged in the insertion section (1) in a plane which is perpendicular to the viewing direction (x), n-times and m-times respectively in a rotationally symmetrical manner with respect to a viewing direction axis (x) of the picture sensor (9).

**Revendications**

1. Instrument médical endoscopique avec une partie d'introduction oblongue distale (1) pour une introduction invasive minimale dans un corps humain ou animal, la partie d'introduction (1) comprenant au moins une première LED (5), une deuxième LED (7) et un capteur d'image (9), la première LED (5), la deuxième LED (7) et le capteur d'image (9) étant orientés dans une direction de regard (x) commune,
la première LED (5) ayant un premier spectre lumineux (19) adapté à l'endoscopie à fluorescence et la deuxième LED (7) ayant un deuxième spectre lumineux (21) adapté à l'endoscopie à la lumière blanche, un filtre de lumière (23) étant disposé, dans la direction de regard (x), devant la deuxième LED (7), le filtre de lumière (23) ayant un spectre de transmission (25), la deuxième LED (7) étant configurée pour éclairer en moyenne, selon le deuxième spectre lumineux (21), de façon plus intense dans une première plage de longueurs d'onde (K) que dans une deuxième plage de longueurs d'onde (L), et le filtre de lumière (23) étant configuré pour laisser passer de la lumière émise par la deuxième LED (7) en moyenne moins dans la deuxième plage de longueurs d'onde (L) que dans la première plage de longueurs d'onde (K) selon le spectre de transmission (25), **caractérisé en ce que** la première LED (5) est disposée, par rapport à la deuxième LED (7), dans la direction de regard (x), davantage en avant.

2. Instrument médical endoscopique selon la revendication 1, la première LED (5), la deuxième LED (7) et le capteur d'image (9) étant disposés à une paroi commune (13) de la partie d'introduction (1).

3. Instrument médical endoscopique selon la revendication 1 ou 2, la première LED (5), la deuxième LED (7) et le capteur d'image (9) étant disposés à la face frontale distale (3) de la partie d'introduction (1) et la direction de regard (x) passant de manière distale dans la direction longitudinale de la partie d'introduction (1).

4. Instrument médical endoscopique selon l'une des revendications précédentes, le filtre de lumière (23) étant un filtre d'absorption.

5. Instrument médical endoscopique selon l'une des revendications précédentes, le filtre de lumière (23) étant un filtre de suppression d'infrarouge, la première plage de longueurs d'onde (K) se trouvant dans le spectre lumineux visible en-dessous de 550 nm et la deuxième plage de longueurs d'onde (L) dans le spectre lumineux visible au-dessus de 550 nm.

6. Instrument médical endoscopique selon l'une des revendications précédentes, le capteur d'image (9) ayant, sur un plan vertical par rapport à la direction de regard (x), sensiblement la même distance (C) de la première LED (5) que de la deuxième LED (7) et étant disposé de préférence entre la première LED (5) et la deuxième LED (7).

7. Instrument médical endoscopique selon l'une des revendications précédentes, le premier spectre lumineux (19) et le deuxième spectre lumineux (21) étant différents ou sensiblement identiques.

8. Instrument médical endoscopique selon l'une des revendications précédentes, la première LED (5) étant une LED diffusant une lumière blanche et la deuxième LED (7) étant une LED diffusant une lumière bleue, ou la première LED (5) et la deuxième LED (7) étant du même type.

9. Instrument médical endoscopique selon l'une des revendications précédentes, le filtre de lumière (23) comprenant un côté d'admission de lumière (27) et un côté de sortie de lumière (29) et ayant une épaisseur (D) entre le côté d'admission de lumière (23) et le côté de sortie de lumière (29) dans la direction de regard (x) et un diamètre (B) de manière orthogonale à la direction de regard (x), l'épaisseur allant de 0,3 mm jusqu'à 80% du diamètre.

10. Instrument médical endoscopique selon l'une des revendications précédentes, le filtre de lumière (23) comprenant un côté d'admission de lumière (27) et un côté de sortie de lumière (29) et ayant une épaisseur de 0,3 mm à 1,2 mm entre le côté d'admission de lumière (27) et le côté de sortie de lumière (29) dans la direction de regard (x).

11. Instrument médical endoscopique selon l'une des revendications précédentes, la distance (H) entre le côté d'admission de lumière (31) du filtre de lumière (23) et le côté de sortie de lumière (31) du deuxième LED (7) étant de moins que 30%, de préférence de moins de 10% de l'épaisseur (D) du filtre de lumière (23) dans la direction de regard (x).

12. Instrument médical endoscopique selon l'une des revendications précédentes, le filtre de lumière (23) et la deuxième LED (7) étant disposés dans un évidement (11b) dans une paroi (13) de la partie d'introduction, la paroi (13) définissant une surface extérieure (15) et la distance (S) entre le côté de sortie de lumière (31) de la deuxième LED (7) et la surface extérieure (15) étant tout au plus des deux tiers du diamètre (B) de l'évidement (11b).

13. Instrument médical endoscopique selon l'une des revendications précédentes, le deuxième spectre lumineux (21) de la deuxième LED (7) et le spectre de transmission (25) du filtre de lumière (23) diminuant avec une longueur d'onde croissante dans la deuxième zone de longueur d'ondes (L) entre 550 nm et 700 nm, et la transmission du filtre de lumière (23) pour de la lumière émise par la deuxième LED (7) avec une longueur d'onde de 600 nm étant comprise entre 20% et 45%.

14. Instrument médical endoscopique selon l'une des revendications précédentes, au moins un élément de protection (17a,b) transparent à la lumière blanche étant disposé, dans la direction de regard (x), devant un côté de sortie de lumière de la première LED (5) et/ou un côté de sortie de lumière (29) du filtre de lumière (23), l'épaisseur de l'élément de protection (17a,b), dans la direction de regard (x), étant moindre que l'épaisseur (D) du filtre de lumière (23) dans la direction de regard (x).

15. Instrument médical endoscopique selon la revendication 14, ledit du moins un élément de protection (17a,b) se trouvant en contact direct avec le côté de sortie de lumière de la première LED (5) ou encore avec le côté de sortie de lumière (29) du filtre de lumière (23) ou une distance entre ledit au moins un élément de protection (17a,b) et le côté de sortie de lumière de la première LED (5) ou encore le côté de sortie de lumière (29) du filtre de lumière (23) étant de moins de 10% de l'épaisseur (D) du filtre de lumière dans la direction de regard (x).

16. Instrument médical endoscopique selon la revendication 15, entre ledit au moins un élément de protection (17a,b) et le côté de sortie de lumière de la première LED (5) ou bien le côté de sortie de lumière (29) du filtre de lumière (23), un milieu étant disposé qui présente un index de réfraction qui se trouve aux environs de 90% à 110% de l'indice de réfraction du filtre de lumière (23).

17. Instrument médical endoscopique selon la revendication 15 ou 16, ledit au moins un élément de protection (17a,b) étant un verre de protection, une matière synthétique de protection et/ou une couche de dioxyde de silicium déposée sur la première LED ou le filtre de lumière (23)

18. Instrument médical endoscopique selon l'une des revendications précédentes, le filtre de lumière (23) et/ou la deuxième LED (7) étant entouré d'une surface interne de corps de cylindre réfléchissante qui s'étend principalement dans la direction de regard (x).

19. Instrument médical endoscopique selon la revendication 18, la surface interne de corps de cylindre réfléchissante étant constituée par un évidement (11a,b) dans une paroi (13) de la partie d'introduction (1).

20. Instrument médical endoscopique selon l'une des revendications précédentes, une quantité de n ≥ 2 premières LED (5a-c) et/ou une quantité de m ≥ 2 deuxièmes LED (7a-c) étant disposées, dans un plan vertical par rapport à la direction de regard (x), respectivement en une quantité n ou en une quantité m, à symétrie de rotation par rapport à un axe de direction de regard (x) du capteur d'images (9), dans la partie d'introduction (1).

Fig. 1

Fig. 3

Fig. 2

EP 3 752 044 B1

Fig. 4

Fig. 5

Fig. 6

Fig. 7

EP 3 752 044 B1

Fig. 9

Fig. 11

Fig. 8

Fig. 10

18

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9517845 A
- US 20130012815 A1

- WO 2012069542 A1